# EUROPEAN PATENT APPLICATION

(11) **EP 1 815 851 A1**
(43) Date of publication of application: **08.08.2007**
(21) Application number: 06002235.7
(22) Date of filing: 03.02.2006
(51) Int. Cl.: A61K 9/51

(54) **Nanoparticles designed for drug delivery**

(71) Applicant: NanoDel Technologies GmbH, 39120 Magdeburg (DE)
(72) Inventor: Ringe, Kerstin, Dr., 39122 Magdeburg (DE); Kubasch, Julia, Dr., 39112 Magdeburg (DE); Radunz, Hans-Eckart, Prof. Dr., 64367 Mühltal (DE)
(74) Representative: Sandmann, Wolfgang

(57) **Abstract**

The present invention is directed to a method of producing nanoparticles by the miniemulsion method, said nanoparticles being made by adding a defined amount of stabilizer to the reaction system. The present invention is further directed to nanoparticles made by this method and their use for the treatment of diseases and conditions, requiring a pharmaceutical agent to cross one or more physiological barriers, in particular the blood-brain barrier.

## Description

The present invention is directed to a method of producing nanoparticles by the miniemulsion method, said nanoparticles being made by adding a defined amount of stabilizer to the reaction system. The present invention is further directed to nanoparticles made by this method and their use for the treatment of diseases and conditions, requiring a pharmaceutical agent to cross one or more physiological barriers, in particular the blood-brain barrier.

Nanoparticles offer new hope for the detection and treatment of several diseases, in particular of cancer. Nanoparticles, thus, hold great potential for many fields of medicine, particularly cancer treatment. The promise of delivering tumor-killing drugs directly to cancerous cells, thus averting the unwanted side-effects of chemotherapy, has generated a lot of interest in nanoparticles among the medical community.

Furthermore, apart from targeted delivery, nanoparticles showed some surprising effects regarding their capability to cross certain barriers of the human and animal body. For example, it was reported in the U.S. Patent Application S.N. 08/203,326 and the parallel International Patent Application No. PCT/EP 95/00724 (corresponding to WO 95/22963) that drugs may be delivered to the body of mammals, particularly to the body of humans, and may be transported across the blood brain barrier by means of nanoparticles to which drugs are complexed (incorporated, adsorbed or absorbed) and which are surrounded by a coating made of an appropriate surfactant. It was taught in both of said applications that the drug may be an immunosuppressive or anticancer agent. As the appropriate surfactant, there were described a number of generally and commercially available surfactants as, for example, Polysorbate 80 or Tween 80.

WO98/56361 also is directed to nanoparticles and it is disclosed that there is no need to apply an additional coating which surrounds the nanoparticle/drug complex as taught by WO 95/22963.

According to the above prior art, nanoparticles were prepared by what is generically termed as an "in-solvent emulsification-evaporation technique" using single (oil-in-water) or multiple emulsifications (water-in-oil-in-water) depending upon whether the incorporated pharmaceutical agent is hydrophobic or hydrophilic.

After forming the nanoparticles, the solvent is evaporated from the emulsion. The nanoparticles are separated from the remainder by centrifugation, or preferably ultracentrifugation (120,000 to 145,000 g), washed with water, and re-centrifuged and decanted.

From the prior art it is also known to carry out conversions to polymers in miniemulsions. Miniemulsions are dispersions of, e.g., water, an oil phase, and one or more surfactants which have a droplet size of from about 50 to 500 nm. The miniemulsions were considered metastable (cf. Emulsion Polymerization and Emulsion Polymers, Editors P. A. Lovell and Mohamed S. El-Aasser, John Wiley and Sons, Chichester, New York, Weinheim, 1997, pages 700 et seq.; Mohamed S. El-Aasser, Advances in Emulsion Polymerization and Latex Technology, 30th Annual Short Course, Volume 3, Jun. 7-11, 1999, Emulsion Polymers Institute, Lehigh University, Bethlehem, Pa., USA). These dispersions find broad application in the art in cleaning products, cosmetics or body care products.

The preparation of aqueous primary dispersions by means of the free-radical miniemulsion polymerization of olefinically unsaturated monomers is known for example from International Patent Application WO 98/02466 or from German Patents DE-A-196 28 143 and DE-A-196 28 142. In the case of these known processes the monomers can be copolymerized in the presence of different low molecular mass, oligomeric or polymeric hydrophobic substances.

A preferred method of nanoparticle formation is the miniemulsion technique as developed by K. Landfester et al:

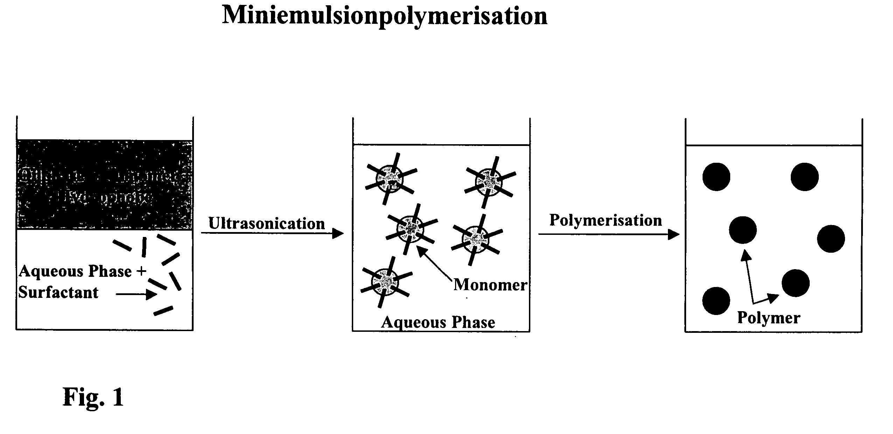

Here, miniemulsions are defined as dispersions of critically stabilized oil droplets with a size between 50 and 500 nm prepared by shearing a system containing water, a surfactant (or "stabilizer") and a hydrophobe. Polymerizations of monomers in such miniemulsions result in particles which have about the same size as the initial droplets. The principle of miniemulsion polymerization is schematically shown in Figure 1 (K. Landfester, Polyreactions in miniemulsions, Macromol. Rapid Comm. 2001, 896-936. K. Landfester, N. Bechthold, F. Tiarks, and M. Antonietti, Formulation and stability mechanisms of polymerizable miniemulsions. Macromolecules 1999, 32, 5222. K. Landfester, Recent Developments in Miniemulsions - Formation and Stability Mechanisms. Macromol. Symp. 2000, 150, 171).

WO0209862 (DE 10101892) discloses a method for producing polymer capsules, pellets or droplets containing an active ingredient or substance, by means of in-situ encapsulation of said active ingredient or substance using a non-radical miniemulsion polymerisation, preferably polyaddition, of suitable monomers. The polymer vehicle system containing the active ingredient or substance, produced by the above method, can be used as a delivery system, in particular, in the field of cosmetics and bodycare, in pharmaceuticals, in adhesive processing and/or in the field of washing and cleaning agents.

DE19852784 discloses a process for stabilizing oil/water micro- or mini-emulsions with the aid of a surfactant (S1) and a co-surfactant (S2), in which (S2) consists of water-insoluble compound(s) selected to give an osmotically stabilized emulsion, other than hexadecane, cetyl alcohol, dodecylmercaptan, long-chain alkyl methacrylates and the dyestuff Blue 70. It further discloses osmotically stabilized micro- or mini-emulsions containing an oil phase, an aqueous phase, a surfactant (S 1) and an ultra-hydrophobic co-surfactant (S2) as defined above.

WO2004017945 discloses the use of nanoparticles for a transfection of DNA into eucaryotic cells. It further discloses the DNA administration to a target organ in the human or animal body. In particular, WO2004017945 teaches the use of nanoparticles for the administration of cancer treatment-related DNA to a tumor-affected target organ in the human or animal body as, for example, the brain in the case of brain tumors. WO2004017945 also discloses to use substances ("stabilizers") which act as enhancers of the bond between the DNA and the nanoparticles. Among others, Diethylaminoethyl-(DEAE)-dextran and dextran 70.000 are listed as stabilizers.

However, the only way for manufacturing those nanoparticles according to WO2004017945 is by conventional emulsion polymerization, i.e. forming nanoparticles directly from a solution of the monomers in a solvent.

It is an object of the present invention to provide an improved method for the manufacture of drug delivery vehicles, which vehicles can be obtained in high yield and solid content. Furthermore, it is an object of the present invention to provide a method for the manufacture of drug delivery vehicles, which is suitable for the large-scale production of said vehicles. Furthermore, it is an object of the invention to provide a delivery vehicle for a pharmaceutical agent which can be specifically adapted to desired release characteristics. It is in particular an object of the invention to provide a delivery vehicle which is suitable to be used for varying medical conditions and which is able to specifically cross the major physiological or biological barriers of the animal or human body and which subsequently is showing modified release characteristics such as sustained release or prolonged release of a pharmaceutical agent in the target tissue. It is an additional object of the present invention to avoid the use of surfactants as, for example, polysorbat 80, as a coating on the nanoparticles.

These objects are achieved by the subject-matter of the independent claims. Preferred embodiments are set forth in the dependent claims.

As a general remark, the nanoparticles of the present invention are formed by a "miniemulsion method", which is per se well known and is, for example, disclosed in K. Landfester, "Polyreactions in miniemulsions", Macromol. Rapid Comm. 2001, 896-936. K. Landfester, N. Bechthold, F. Tiarks, and M. Antonietti, "Formulation and stability mechanisms of polymerizable miniemulsions". This technology basically differs from the well known conventional methods (as, for example, used in WO2004017945) by involving a different order of manufacturing steps: in this approach, a miniemulsion, containing dispersed hydrophobic, monomer containing droplets in a hydrophilic continuous phase, are formed to polymeric particles. In the conventional approach, the polymers are directly formed from the solution containing monomers.

Thus, the term "miniemulsion" as used herein generally means a technology, by which a dispersion is made containing a continuous W (hydrophilic) and an O (hydrophobic) phase, dispersed in the continuous phase. Further, this term is directed to emulsion droplets formed of 1 to 1.000 nm, usually approximately between 50 and 500 nm (the size of the particles following polymerization is identical or almost identical).

A further difference between the conventional process and the miniemulsion process is that in the latter, two liquid phases are brought into contact in the beginning of the method, and an emulsion is formed subsequently. In the conventional emulsion method, only droplets of the one phase are dropped into the other phase in the beginning.

One of the major drawbacks of the conventional approach can be seen in the fact that only suspensions containing about 1% of nanoparticles (solid content) may be obtained therewith. In contrast, the present miniemulsion approach allows a solid content of nanoparticles of even 15-24% or more based on a monomer content of 25% w/w (based on the overall weight of the O/W reaction system). Thus, the yield of solid nanoparticles is dramatically increased and corresponds to about 60-96% of the employed monomers.

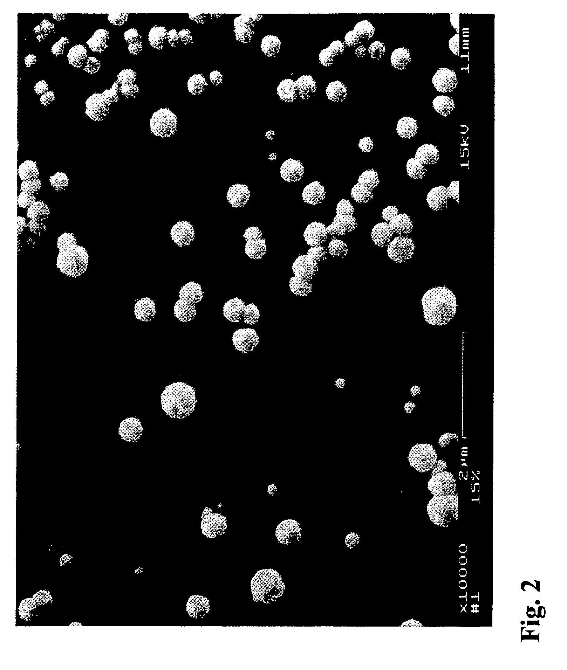

With the present method, it is possible to obtain nanoparticle suspensions of high solid content (range: 15%-24%) and thus in a high yield (60-96%), which is very important in terms of up-scaling. The process is reproducible. The mean diameter of the generated nanoparticles is 200-300 nm (range < 1 µm) (see Figure 2).

As an example, if the O phase is consisting of 6 g monomer + 250 mg hydrophobe, the W phase is 24 g 0.01 N HCl + stabilizer, the maximum content of solids will be 25% w/w (6/24 x 100) corresponding to a yield of 100%. A content of 20% w/w then corresponds to a yield of 80%. In this case, the remaining 20% are represented by monomers, which were lost during polymerization, for example by polymerizing at the vessel surface or at the homogenizer or the like.

The main difference between the present invention and the prior art approaches, however, resides in the fact that no surfactant coating is used for the nanoparticles according to this invention. It surprisingly turned out that the well-known surfactant coatings might have a deleterious influences on the therapeutical safety of the nanoparticles, if those were carrying one or more drugs. In particular, experimental results showed that up to 40 weight-% of the originally added drug (adsorbed to the nanoparticles) were displaced by the surfactant coating. This is, however, leading to an unacceptably varying and inprecise amount of drug bound to the nanoparticles, lowering the therapeutical efficacy and reliability of the drug coated nanoparticles.

Omitting the surfactant coating of the nanoparticles, however, leads to a decreased capability of these nanoparticles to cross physiological barriers, for example the blood-brain-barrier. This can be explained by the fact that the surfactant coating material (e.g. Tween 80) is capable of absorbing ApoE and ApoA, respectively, present in blood, thereby allowing a presentation of the nanoparticles to lipoprotein receptors of the blood-brain barrier. This in turn is leading to receptor mediated endocytosis of the nanoparticles and, thus, to a passage of the nanoparticles through the blood-brain-barrier.

Therefore, it would be disadvantageous to simply remove the surfactant coating in order to avoid displacement of the drugs adsorbed on the nanoparticles.

The inventors now found a solution to overcome both problems, i.e. to avoid the drawback which is related to the use of a surfactant coating and to maintain the coating's positive effects on crossing physiological barriers, in particular the blood-brain-barrier:

The same or similar surfactant to those forming the prior art surfactant coatings should be incorporated into the nanoparticles already in their process of manufacture. More precisely, the surfactants or stabilizers were found to be effective if they were incorporated in an amount of between 10-25 weight% based on the overall weight of the polymerizable monomers into the nanoparticles. The method of the present invention, thus, considerably simplifies the fabrication procedure and facilitates clinical application as mixing with a surfactant is no longer required prior to application to the organism.

Thus, in a first aspect, the method of producing nanoparticles according to the present invention comprises the steps of:
a) providing a reaction system comprising O and W type liquid phases, one or more stabilizers and polymerizable monomers, wherein the one or more stabilizers are added in an amount of between 10-25 weight% based on the overall weight of the polymerizable monomers,
b) forming an O/W type miniemulsion, the O-phase droplets containing said monomers,
c) polymerizing said monomers in order to form nanoparticles, wherein one or more pharmaceutical agents capable of being bound to said nanoparticles are added to said nanoparticles, thereby producing nanoparticles coated by said one or more pharmaceutical agents, and/or, wherein one or more pharmaceutical agents are added in step a).

The amount of the one or more stabilizers is between 10-25 weight% based on the overall weight of the polymerizable monomers, i.e. is not including the values of 10 and 25 weight%. It should be further noted that the "amount of stabilizer" as used herein also includes the overall amount of several combined stabilizers. For example, said amount of between 10 and 25 % by weight might be composed of 10 % of sodium dodecylsulfate and 10% Polysorbate 80 (see Example 2).

The term "stabilizer" as used in this invention shall comprise any substance capable of stabilizing an emulsion. Those substances are preferably surface active substances, i.e. surfactants.

In a further aspect, the invention provides a method of producing nanoparticles which consists of the steps mentioned above.

The inventors found that the broadest possible range of stabilizers or surfactants to be incorporated into the nanoparticles of the present invention is between 10-25 weight% based on the overall weight of the polymerizable monomers. This range is true for all stabilizers (surfactants) tested. This range can be explained by experimental results indicating that an amount of 10 weight% and less based on the overall weight of the polymerizable monomers is not sufficient to allow the nanoparticles to cross the physiological barrier in a sufficient amount. If, on the other hand, 25 weight% or more based on the overall weight of the polymerizable monomers will be used, an unacceptable high amount of stabilizers (surfactants) will be delivered to the body of the patient.

The term "reaction system" as used herein is defined as any environment, which fulfills the requirements of forming a miniemulsion according to the above definition.

As mentioned above, the already known miniemulsion technique as, for example, disclosed in Landfester et al. (supra), may be used to prepare the miniemulsion. This may, for example be done by at first combining monomers, an O and a W phase and a stabilizer (defined below). It is noted that the W phase may contain further ingredients, for example HCl for setting a suitable pH value. Second, the reaction system may be mixed by, for example, a homogenizer or the like, in order to mix all ingredients.

The preparation of the miniemulsion itself is performed by applying high shear forces to the reaction system, for example by ultrasound and high pressure homogenizers. Furthermore, the shear forces may be applied for a time range of from 2-5 min. depending on the size of the reaction system and the homogenizer used. Basically, a time range of between 3 and 4 min. is regarded as being sufficient.

The ultrasound homogenizer may have an amplitude of about 60-100%, preferably about 70-90%.

The temperatures used in this process are preferably from -1 to 5 °C, preferably 0°C.

Generally the weight ratio of O to W phase is from 15-40 % w/w, preferably 20-30 % w/w and more preferably about 25 % w/w.

Following preparing the miniemulsion, the polymerisation process is started, for example by simply increasing the pH to a value of pH 7,0 or the like. This may be done, whenever PBCA monomers are used in the reaction system and the pH increase is performed, for example, by adding a sufficient amount of K₂CO₃ to the reaction system.

The nanoparticles of the invention preferably have a diameter in the range of between 50 and 500 nm, more preferably between 200 and 300 nm (see Fig. 2 as an example).

In the present invention, it surprisingly turned out that the nanoparticles prepared by the above miniemulsion method have unexpected characteristics and advantages. At first, as mentioned above, the yield of the manufacturing method is much higher than that of the conventional ones. Furthermore, however, it turned out that the nanoparticles produced by the method of the present invention have an improved capability to bind pharmaceutical agents to their surface. Surprisingly, this effect could be shown independently from the pharmaceutical agent's (or drug's) characteristics involved.

Without being bound to any specific theory, it is assumed that the three-dimensional structure of the nanoparticles of the present invention allows a more stable attachment of the drug molecules to the nanoparticle. Microscopic evaluation showed not a smooth, uniform surface of the nanoparticles, but a clew-like structure of the surface. It is our theory that this clew-like structure facilitates binding of drug molecules to the nanoparticles independently of their physical or chemical behaviour.

However, it should also be mentioned that the one or more pharmaceutical agents do not have to be coated on the nanoparticles necessarily. It is also possible to incorporate said agents into the nanoparticles by adding them already in step a). It depends on the pharmaceutical agent and its chemical and/or physical properties in which of the two phases it will preferably be introduced in this case. It is clear that a lipophilic agent preferably is incorporated in the O type liquid phase, a hydrophilic agent will be introduced into a W type liquid phase. Also a combination of both approaches is possible, i.e. adding a pharmaceutical agent in step a) and coating the same on the nanoparticles following preparation.

In a preferred embodiment, the stabilizer is added in step a) in an amount of 12 to 23, more preferably 15 or 20 weight% based on the overall weight of the polymerizable monomers.

In a further embodiment, a further step d) is performed wherein to the provided nanoparticles a medium is added allowing the transport of said nanoparticles to a target within or on a mammal after administration. More preferably, step d) provides the nanoparticles in form of a pharmaceutical composition.

Such a pharmaceutical composition comprises a physiologically acceptable carrier and/or diluent allowing the transport of said nanoparticles to the target within a mammal, preferably a human, after administration. Preferably, said carrier and/or diluent is/are selected from the group consisting of water, physiologically acceptable aqueous solutions containing salts and/or buffers and any other solution acceptable for administration to a mammal. Such carriers and diluents are well known to a person skilled in this field and comprise distilled water, de-ionized water, pure or ultrapure water, saline, phosphate buffered saline (PBS), solutions containing usual buffers which are compatible with the other components of the drug targeting system etc.

In order to use the nanoparticles *in vivo,* they may be reconstituted into a suspension with normal saline or phosphate buffered aqueous solution at physiological pH and osmolarity.

Typically, the nanoparticles are present in the injectable suspension at a concentration ranging from 0.1 mg nanoparticles per ml suspending fluid to 100 mg nanoparticles per ml suspending fluid. 10 mg nanoparticles per ml is preferred. The amount of nanoparticles used will strongly depend on the amount of pharmaceutical agent contained in an individual nanoparticle and the skilled artisan or the physician in charge will be readily able to adapt the dosage of the nanoparticles to the specific circumstances.

Alternatively, the pharmaceutical composition may take other forms required to transfer the delivery vehicle of the invention to and across other physiological barriers, for example to and across the blood-air barrier. Then it may, for example have the form of an aerosol or the like in order to deliver the composition by inhalation to the barrier in question.

The pharmaceutical composition preferably takes the form of a medicament for oral, intravenous, intraperitoneal, subcutaneous, intramuscular, intranasal, pulmonal or rectal administration, preferably for the oral or intravenous administration.

In the method of the present invention, the step of coating the nanoparticles with a pharmaceutical agent preferably comprises mixing said nanoparticles with a solution of said one or more pharmaceutical agents and allowing a sufficient time for an effective amount of said one or more pharmaceutical agents to be adsorbed onto and/or absorbed by said nanoparticles.

The O phase used in the present method preferably contains a hydrophobe, selected from olive oil, miglyol and/or hexadecane. The amount of hydrophobe is usually relatively small and should be sufficient to prevent Ostwald ripening (about 2-10% w/w based on the overall weight of the O-phase (further containing the monomer)).

The polymerizable monomers are used in order to form a polymeric material which preferably is selected from the group consisting of polyacrylates, polymethacrylates, polycyanoacrylates, preferably polyalkylcyanoacrylates, polyarylamides, polylactates, polyglycolates, polyanhydrates, polyorthoesters, gelatin, polysaccharides, albumin, polystyrenes, polyvinyls, polyacrolein, polyglutaraldehydes and derivatives, copolymers and mixtures thereof.

Preferred polymeric materials comprise solid or film forming polymers, preferably polyalkylcyanoacrylates, more preferably polybutylcyanoacrylates, polylactic acid and polybutyric acid and mixtures and derivatives thereof.

The polymeric materials used (produced) in the present invention preferably are biodegradable. This terms denotes any synthetic or naturally-derived polymeric material which is known as being suitable for uses in the body of a living being, i.e., is biologically inert and physiologically acceptable, non-toxic, and, in the delivery systems of the present invention, is biodegradable in the environment of use, i.e., can be resorbed by the body.

Further biodegradable polymers which may be also used to formulate nanoparticles include, but are not limited to, polyesters, such as polyglycolides and polylactic polyglycolic copolymers (PLGA); polyethers, such as such as hydroxy-terminated poly(ε-caprolactone)-polyether or polycaprolactone (PCL); polyanhydrides; polyacrylamides; poly(orthoesters); polyamino acids; and biodegradable polyurethanes. It is to be understood that the term polymer is to be construed as to include copolymers and oligomers.

In an embodiment the stabilizer provided in step a) comprises one or more of the following substances:
fatty acid esters of sorbitol, preferably, sorbitan monolaurate, or sorbitan monooleate; poloxamines, preferably poloxamine 904 or 1508; polyoxyethylene ethers and polyoxyethylene esters; sodium lauryl sulfate; preferably sodium dodecylsulfate; preferably polysorbates, more preferably polysorbate 60 and most preferably polysorbate 80; preferably poloxamers, more preferably poloxamer 188, 338 or 407; preferably polyoxyethylene glycols, more preferably Lutensol 50 or 80; and mixtures of two or more of said substances.

It should be noted that the molecular structure of all of these stabilizers (or surfactants) shows similarities, as it is the case for their properties.

The one or more pharmaceutical agents used for coating the nanoparticles are selected from therapeutic agents and diagnostic agents.

The therapeutic agent is preferably selected from substances which are incapable of crossing physiological barrieres without a delivery vehicle or carrier. This physiological barrier is preferably selected from, although not restricted to, the group consisting of blood-brain barrier (bbb), blood-air barrier, blood-cerebrospinal fluid barrier and buccal mucosa. Additionally, the one or more therapeutic agents may have central nervous system activity but cannot cross the blood brain barrier without a delivery vehicle.

It is noted that the terms "drug" and "therapeutic agent" are used interchangeably herein.

According to a preferred embodiment, the delivery vehicle of the invention comprises one or more therapeutic agents selected from the group consisting of drugs acting at synaptic sites and neuroeffector junctional sites; general and local analgetics; hypnotics and sedatives; drugs for the treatment of psychiatric disorders such as depression and schizophrenia; anti-epileptics and anticonvulsants; drugs for the treatment of Parkinson's and Huntington's disease, aging and Alzheimer's disease; excitatory amino acid antagonists, neurotrophic factors and neuroregenerative agents; trophic factors; drugs aimed at the treatment of CNS trauma or stroke; drugs for the treatment of addiction and drug abuse; anti-obesity drugs; antacoids and anti-inflammatory drugs; chemotherapeutic agents for parasitic infections and diseases caused by microbes; immunosuppressive agents and anti-cancer drugs; hormones and hormone antagonists; heavy metals and heavy metal antagonists; antagonists for non-metallic toxic agents; cytostatic agents for the treatment of cancer; diagnostic substances for use in nuclear medicine; immunoactive and immunoreactive agents; transmitters and their respective receptor agonists and receptor antagonists, their respective precursors and metabolites; transporter inhibitors; antibiotics; antispasmodics; antihistamines; antinauseants; relaxants; stimulants; sense and antisense oligonucleotides; cerebral dilators; psychotropics; antimanics; vascular dilators and constrictors; anti-hypertensives; drugs for migraine treatment; hypnotics, hyperglycemic and hypoglycemic agents; anti-asthmatics; antiviral agents, preferably anti HIV agents; genetic material suitable for the DNA or anti-sense treatment of diseases; and mixtures thereof.

The term "DNA"as used in the present specification basically refers to any DNA conceivable in the present field of the art. In preferred embodiments, the term "DNA" is meant to comprise two types of DNA, i. e. plasmid DNA, more preferably plasmid DNA comprising the information of tumor suppressor genes, even more preferably plasmid DNA comprising the information of the tumor suppressor genes p53 and pRB, on the one hand, and antisense oligonucleotides, more preferably antisense oligonucleotides against oncogenes, even more preferably antisense oligonucleotides against oncogenes like Bcl2, on the other hand. There may be used one type of DNA (and, consequently, one type of DNA-loaded nanoparticles) in the present invention. Alternatively, two or more types of DNA may be used, resulting into a plurality of types of nano- particles loaded with different types of DNA and useable in accordance with the present invention.

Surprisingly, DNA and particularly DNA of the above two types could be adsorbed onto nanoparticles, and the resulting DNA-nanoparticle complexes could be inoculated into the organism, particularly into the organism suffering from cancer (specifically, but not limited to, brain cancer). Thereafter, a suppression of the tumor proliferation could be observed, and even a tumor necrosis and apoptosis could be induced.

In a preferred, but not essential embodiment of the invention, plasmid DNA comprising a promoter, more preferably plasmid DNA comprising an inducible promoter, can be loaded onto the nanoparticles. By the novel step to load the DNA containing an inducible promoter onto the nanoparticle and to express it within the cell, an inducible promoter, and thereby an external control of the expression of the relevant gene may be achieved, and the gene may be "switched" on and off at will. As an unexpected advantage over the prior art, the timing of the gene/DNA expression can be controlled. Such a control may reduce toxic side effects of a continuous gene expression and/or may lower the probability that cells become resistant to the gene products, producing a negative selection.

In a preferred embodiment of the invention, the human papilloma virus upstream regulatory region (HPV-URR) was used as the inducible promoter. The expression of the tumor suppressor is induced after the administration of Dexamethasone or other inducers or compounds. In that way, an apoptosis of the tumor cells as well as a regression of the tumor could be achieved. Other exemplary promoters useable in accordance with the present invention are the cytomegalia virus (CMV) promoter or the simian virus 40 (SV 40) promoter.

Furthermore, strong effects including a tumor regression could be achieved by a combination administration of one or more than one type(s) of DNA-containing nanoparticles and nanoparticles containing one or more than one cytostatically effective substance(s).

In a preferred embodiment, tumor suppressor DNA, even more preferred behind an inducible promoter, may be injected prior to inoculation of a nanoparticle complex containing a cytostatically effective compound. In an even more preferred embodiment, the cytostatically effective compound is Doxorubicine.

In the process of transfection of DNA into cells, and in the process of administration of a DNA to a target organ in the human or animal body as well, the first step comprises the preparation of nanoparticles in a way defined above.

For further information it is explicitely referred to WO 2004/017945, and its content is incorporated herein in its entirety.

Further typical active ingredients (e.g., drugs) can be any substance affecting the nervous system or used for diagnostic tests of the nervous system. These are described by Gilman et al. (1990), "Goodman and Gilman's - The Pharmacological Basis of Therapeutics", Pergamon Press, New York, and include the following agents:
acetylcholine and synthetic choline esters, naturally occurring cholinomimetic alkaloids and their synthetic congeners, anticholinesterase agents, ganglionic stimulants, atropine, scopolamine and related antimuscarinic drugs, catecholamines and sympathomimetic drugs, such as epinephrine, norepinephrine and dopamine, adrenergic agonists, adrenergic receptor antagonists, transmitters such as GABA, glycine, glutamate, acetylcholine, dopamine, 5-hydroxytryptamine, and histamine, neuroactive peptides; analgesics and anesthetics such as opioid analgesics and antagonists; preanesthetic and anesthetic medications such as benzodiazepines, barbiturates, antihistamines, phenothiazines and butylphenones; opioids; antiemetics; anticholinergic drugs such as atropine, scopolamine or glycopyrrolate; cocaine; chloral derivatives; ethchlorvynol; glutethimide; methyprylon; meprobamate; paraldehyde; disulfiram; morphine, fentanyl and naloxone; psychiatric drugs such as phenothiazines, thioxanthenes and other heterocyclic compounds (e.g., halperiodol); tricyclic antidepressants such as desimipramine and imipramine; atypical antidepressants (e.g., fluoxetine and trazodone), monoamine oxidase inhibitors such as isocarboxazid; lithium salts; anxiolytics such as chlordiazepoxyd and diazepam; anti-epileptics including hydantoins, anticonvulsant barbiturates, iminostilbines (such as carbamazepine), succinimides, valproic acid, oxazolidinediones and benzodiazepines. Anti-Parkinson drugs such as L-DOPA/CARBIDOPA, apomorphine, amatadine, ergolines, selegeline, ropinorole, bromocriptine mesylate and anticholinergic agents;
antispasticity agents such as baclofen, diazepam and dantrolene; neuroprotective agents, such as excitatory amino acid antagonists, neurotrophic factors and brain derived neurotrophic factor, ciliary neurotrophic factor, or nerve growth factor; neurotrophine (NT) 3 (NT3); NT4 and NT5; gangliosides; neuroregenerative agents; drugs for the treatment of addiction and drug abuse include opioid antagonists and anti-depressants; autocoids and anti-inflammatory drugs such as histamine, bradykinin, kailidin and their respective agonists and antagonists; chemotherapeutic agents for parasitic infections and microbial diseases; anti-cancer drugs including alkylating agents (e.g., nitrosoureas) and antimetabolites; nitrogen mustards, ethylenamines and methylmelamines; alkylsulfonates; folic acid analogs; pyrimidine analogs, purine analogs, vinca alkaloids; anti-obesity drugs like phendimetrazine, phentermine, diethylpropion, sibutramine; and antibiotics.

According to a preferred embodiment, the diagnostic agent is selected from the group consisting of diagnostics useful in the diagnosis in nuclear medicine and in radiation therapy.

The method of the invention in an embodiment also preferably comprises a step, wherein the stabilizer is at least partially removed from the nanoparticles following step c). However, the range of between 10-25 weight% as indicated in step a) of the present invention should be observed.

The additional step can preferably be done by dialysis or centrifugation. It surprisingly turned out that, although required in the method of manufacture per se, the stabilizers may be at least partially removed after the final nanoparticles were formed. In other words, an "excess" of stabilizers can be removed which is not required for maintaining the stability of the nanoparticles, but can cause potential risks for *in vivo* applications. It is assumed that the lowest possible amount of stabilizers in the nanoparticles should be regarded as having the lowest *in vivo* risk. However, the amount may not be too low since that would hamper the nanoparticle's ability to cross the physiological barriers.

In a second aspect, the present invention provides coated nanoparticles or a pharmaceutical composition obtainable by the method as defined above.

Said coated nanoparticles or the pharmaceutical composition may be used for the manufacture of a medicament for the treatment of diseases and conditions, requiring a pharmaceutical agent to cross one or more physiological barriers, in particular the blood-brain barrier.

All publications, patent applications, patents, and other references mentioned herein are incorporated by reference in their entirety. In case of conflict, the present specification, including definitions, will control. In addition, the materials, methods, and examples are illustrative only and not intended to be limiting.

The invention is now further illustrated by the accompanying drawings, in which:
Fig. 1 is an illustration showing the miniemulsion polymerization technique;
Fig. 2 is a photograph of nanoparticles manufactured in accordance with the present invention;

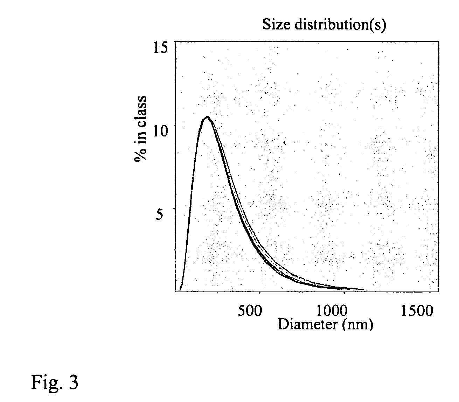

Fig. 3 is a representation of the particle size distribution of nanoparticles manufactured in Example 1;

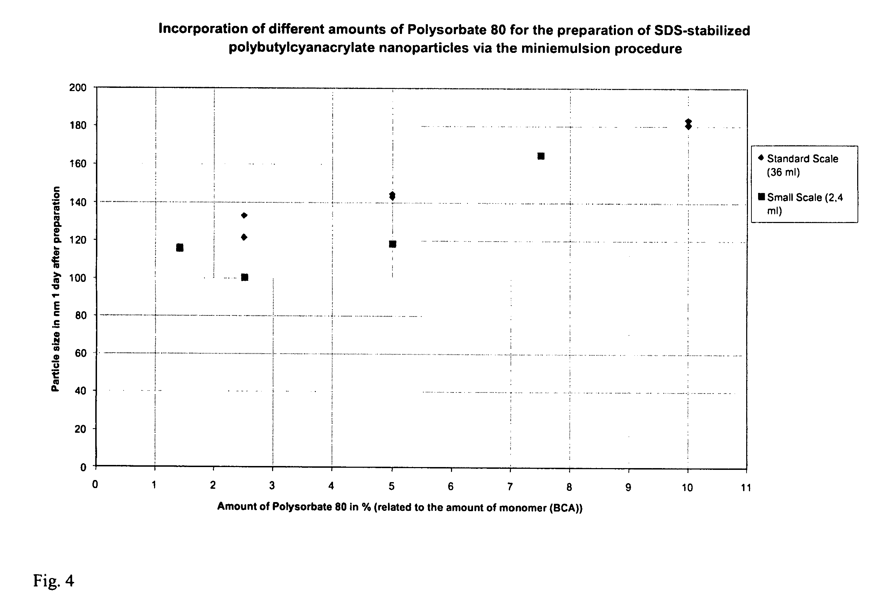

Fig. 4 shows different amounts of Polysorbate 80 (1,4 %, 2,5 %, 5 %, 7,5 % and 10 %) incorporated into polybutylcyanoacrylate nanoparticles stabilized by sodium dodecylsulfate (SDS). The particle size of the resultant nanoparticle suspension was measured by photon correlation spectroscopy. The particle size of the obtained nanoparticle suspension in reliance to the incorporated amount of Polysorbate 80 is shown;
Fig. 5 depicts cryostatic slides of rat brain tissue after intraveneous injection of pure FITC-labelled nanoparticles (not according to the invention);
Fig. 6 shows cryostatic slides of rat brain tissue after intravenous injection of FITC-labelled SDS-nanoparticles having incorporated Polysorbate 80 (according to the invention).

### Examples:

### Example 1:

### Incorporation of Polysorbate 80 into SDS-stabilized Polybutylcyanacrylate Nanoparticles using the Miniemulsion Method.

Two separate solutions are prepared.
Solution 1: 2,4 ml of hydrochloric acid (0,1 mol·l⁻¹) are added into a 10 ml flask (PP). Then 0,06 g sodium dodecylsulfate (SDS) and 30 mg of polysorbate 80 are added to this solution. The resulting solution is stirred until a clear solution is formed.
Solution 2: 32,3 µL hexadecane and 545,5 µL (0,6 g) *n*-butyl-α-cyanacrylate (Indermil) are added into a 5 ml flask. The flask is pivoted until a homogeneous solution is formed.

Solution 2 is added to solution 1 and the miniemulsion is immediately formed by ultrasonication (amplitude 70%, 0°C) of the resulting suspension for 2 min. Then the polymerisation of the monomer droplets is initiated by rapidly changing the pH value from 1 to 7 by adding the miniemulsion into 2,4 ml of 0,1 mol l⁻¹ aqueous NaOH solution (10 ml flask). The obtained suspension is then stirred for 5 min. The nanoparticle suspension possesses a solid content of 111,8 mg/ml and a mean particle size (photon correlation spectroscopy) of 118,4 ± 0,7 nm.

### Example 2:

### Incorporation of Polysorbate 80 and FITC-Dextran into SDS-stabilized Polybutylcyanacrylate Nanoparticles using the Miniemulsion Method.

Two separate solutions are prepared.
Solution 1: 2,4 ml of Hydrochloric acid (0,1 mol·l⁻¹) are added into a 10 ml flask (PP). Then 0,06 g Sodiumdodecylsulfate (SDS), 60 mg of Polysorbate 80 and 9,0 mg Fluoresceinisothiocyanat-Dextran 77.000 (FITC-Dextran) are added to this solution. The resulting solution is stirred until a clear solution is formed.
Solution 2: 32,3 µL Hexadecane and 545,5 µL (0,6 g) *n*-Butyl-α-cyanacrylate (Indermil) are added into a 5 ml flask. The flask is pivoted until a homogeneous solution is formed.

Solution 2 is added to solution 1 and the miniemulsion is immediately formed by ultrasonication (amplitude 70%, 0°C) of the resulting suspension for 2 min. Then the polymerisation of the monomer droplets is initiated by rapidly changing the pH value from 1 to 7 by adding the miniemulsion into 2,4 ml of 0,1 mol 1⁻¹ aqueous NaOH solution (10 ml flask). The obtained suspension is then stirred for 5 min. The resulting labelled nanoparticles are then dialysed (10 times) to remove possible unbound FITC-Dextran and excess of stabilizers. The nanoparticle suspension possesses a solid content of 28,9 mg/ml and a mean particle size (photon correlation spectroscopy) of 258,4 ± 7,0 nm.

The obtained nanoparticle suspension is then diluted with 0,9 % NaCl-solution (1:1) for the in vivo experiments.

### Example 3:

### Incorporation of FITC-Dextran into SDS-stabilized Polybutylcyanacrylate Nanoparticles using the Miniemulsion Method (Comparative Example).

Two separate solutions are prepared.
Solution 1: 2,4 ml of Hydrochloric acid (0,1 mol·l⁻¹) are added into a 10 ml flask (PP). Then 0,06 g Sodiumdodecylsulfate (SDS) and 9,0 mg Fluoresceinisothiocyanat-Dextran 77.000 (FITC-Dextran) are added to this solution. The resulting solution is stirred until a clear solution is formed.
Solution 2: 32,3 µL Hexadecane and 545,5 µL (0,6 g) *n*-Butyl-α-cyanacrylate (Indermil) are added into a 5 ml flask. The flask is pivoted until a homogeneous solution is formed.

Solution 2 is added to solution 1 and the miniemulsion is immediately formed by ultrasonication (amplitude 70%, 0°C) of the resulting suspension for 2 min. Then the polymerisation of the monomer droplets is initiated by rapidly changing the pH value from 1 to 7 by adding the miniemulsion into 2,4 ml of 0,1 mol 1⁻¹ aqueous NaOH solution (10 ml flask). The obtained suspension is then stirred for 5 min. The resulting labelled nanoparticles are then dialysed (10 times) to remove possible unbound FITC-Dextran and excess of stabilizer. The nanoparticle suspension possesses a solid content of 25,2 mg/ml and a mean particle size (photon correlation spectroscopy) of 230,9 ± 9,8 nm.

The obtained nanoparticle suspension is then diluted with 0,9 % NaCl-solution (1:1) for the in vivo experiments.

### Example 4:

### In Vivo Experiments

Number of rats per group: 2
Group 1: intravenous injection of 500 µl of pure FITC-labelled SDS-nanoparticles (Example 3)
Group 2: intravenous injection of 500 µl of FITC-labelled SDS-nanoparticles having incorporated Polysorbate 80 (Example 2)

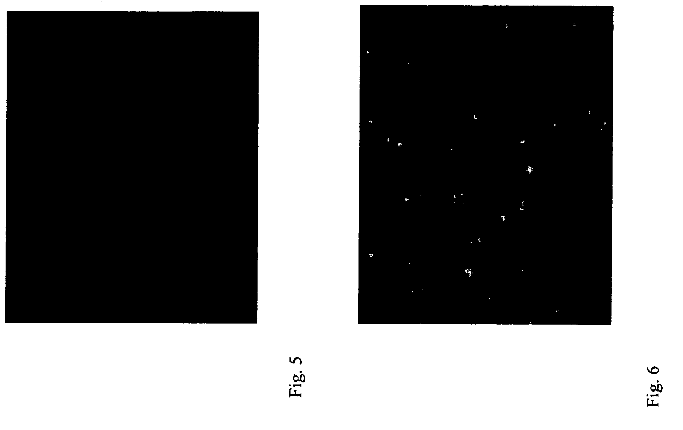

45 min. after the intravenous injection of the labelled nanoparticles the rats were anesthetized using Isofluran and killed by neck fracture. The brain was removed and kept in liquid nitrogen. Cryostatic slices (15 µm) were prepared and examined via fluorescence microscopy (see Fig. 5 and 6).

### Results:

Fig. 5 shows rat brain tissue after the i.v.- injection of pure FITC-labelled SDS-nanoparticles. The brain tissue doesn't reveal any fluorescence derived by the transport of the labelled nanoparticles into the brain. In contrast Fig. 6 demonstrates a significant fluorescence in the brain tissue of rats after the i.v. - injection of FITC-labelled SDS-nanoparticles having incorporated Polysorbate 80. It can be concluded that the incorporation of a specified amount of stabilizer (in this case Polysorbate 80) during the fabrication process of the nanoparticles leads to a significant transport of these nanoparticles across the blood brain barrier into the brain of rats which nanoparticles do not require a surfactant coating.

## Claims

1. A method of producing nanoparticles comprising one or more pharmaceutical agents, having the steps of:
a) providing a reaction system comprising O and W type liquid phases, one or more stabilizers and polymerizable monomers, wherein the one or more stabilizers are added in an amount of between 10-25 weight% based on the overall weight of the polymerizable monomers,
b) forming an O/W type miniemulsion, the O-phase droplets containing said monomers,
c) polymerizing said monomers in order to form nanoparticles, wherein one or more pharmaceutical agents capable of being bound to said nanoparticles are added to said nanoparticles, thereby producing nanoparticles coated by said one or more pharmaceutical agents, or, wherein one or more pharmaceutical agents are added in step a).

2. The method of claim 1, wherein the one or more stabilizer are added in step a) in an amount of 12 to 23, preferably 15 or 20 weight% based on the overall weight of the polymerizable monomers.

3. The method of claim 1 or 2, wherein a further step d) is performed wherein to the nanoparticles a medium is added allowing the transport of said nanoparticles to a target within or on a mammal after administration.

4. The method of claim 3, wherein step d) provides nanoparticles in form of a pharmaceutical composition which preferably takes the form of a medicament for oral, intravenous, intraperitoneal, subcutaneous, intramuscular, intranasal, pulmonal or rectal administration, more preferably for the oral or intravenous administration.

5. The method of one or more of the preceding claims, wherein the step of coating said nanoparticles with said one or more pharmaceutical agents comprises mixing said nanoparticles with a solution of said one or more pharmaceutical agents and allowing a sufficient time for an effective amount of said one or more pharmaceutical agents to be adsorbed onto and/or absorbed by said nanoparticles.

6. The method of one or more of the preceding claims, wherein the O phase contains olive oil, miglyol and/or hexadecane.

7. The method of one or more of the preceding claims, wherein the polymeric material is selected from the group consisting of polyacrylates, polymethacrylates, polycyanoacrylates, preferably polyalkylcyanoacrylates, polyarylamides, polylactates, polyglycolates, polyanhydrates, polyorthoesters, gelatin, polysaccharides, albumin, polystyrenes, polyvinyls, polyacrolein, polyglutaraldehydes and derivatives, copolymers and mixtures thereof, and/or
wherein the stabilizer provided in step a)comprises one or more of the following substances:
fatty acid esters of sorbitol, preferably, sorbitan monolaurate, or sorbitan monooleate; poloxamines, preferably poloxamine 904 or 1508; polyoxyethylene ethers and
polyoxyethylene esters; sodium lauryl sulfate; sodium dodecylsulfate; preferably polysorbates, more preferably polysorbate 60 and most preferably polysorbate 80; preferably poloxamers, more preferably poloxamer 188, 338 or 407; preferably polyoxyethylene glycols, more preferably Lutensol 50 or 80; and mixtures of two or more of said substances.

8. The method of one or more of the preceding claims, wherein the one or more pharmaceutical agents are selected from therapeutic agents and diagnostic agents, wherein the therapeutic agent preferably is selected from therapeutic agents having central nervous system activity but cannot cross the blood brain barrier without a delivery vehicle, and wherein the diagnostic agent is selected from the group consisting of diagnostics useful in the diagnosis in nuclear medicine and in radiation therapy.

9. The method of one or more of the preceding claims, wherein the stabilizer is at least partially removed from the nanoparticles following the method of producing nanoparticles, wherein the removal preferably is done by means of dialysis or centrifugation.

10. Nanoparticles or a pharmaceutical composition obtainable by the method of one or more of claims 1-9.

11. Use of the nanoparticles or the pharmaceutical composition of claim 10 for the manufacture of a medicament for the treatment of diseases and conditions, requiring a pharmaceutical agent to cross one or more physiological barriers, in particular the blood-brain barrier.
